**Europäisches Patentamt**

**European Patent Office**

**Office européen des brevets**

(19)

(11) Veröffentlichungsnummer: **0 178 445**
**B1**

## EUROPÄISCHE PATENTSCHRIFT

(12)

(45) Veröffentlichungstag der Patentschrift:
**17.02.88**

(51) Int. Cl.⁴: **A 61 F 2/38**

(21) Anmeldenummer: **85111231.8**

(22) Anmeldetag: **05.09.85**

(54) Kniegelenk-Endoprothese.

(30) Priorität: **11.09.84 DE 3433263**

(43) Veröffentlichungstag der Anmeldung:
**23.04.86 Patentblatt 86/17**

(45) Bekanntmachung des Hinweises auf die Patenterteilung:
**17.02.88 Patentblatt 88/7**

(84) Benannte Vertragsstaaten:
**AT BE CH DE FR GB IT LI LU NL SE**

(56) Entgegenhaltungen:
**DE - A - 2 549 819**
**DE - A - 2 728 427**
**DE - A - 2 744 710**

(73) Patentinhaber: **S + G IMPLANTS GMBH,**
**Grapengiesserstrasse 21, D-2400 Lübeck (DE)**

(72) Erfinder: **Grundel, Hans, Gärtnergasse 4, D-2400 Lübeck (DE)**

(74) Vertreter: **Wilcken, Thomas, Dipl.-Ing. et al,**
**Musterbahn 1, D-2400 Lübeck (DE)**

ACTORUM AG

## Beschreibung

Die Erfindung bezieht sich auf eine Kniegelenk-Endoprothese mit den Merkmalen nach dem Oberbegriff des Anspruches 1.

Bei Kniegelenk-Endoprothesen der vorerwähnten Art (DE-A-2 549 819) legt sich in der Strecklage des Gelenkes die vordere Überbrückung der beiden Kufen des Femurteiles gegen eine obere vordere Nase des Mittelsteges, und die Enden des Gelenkzapfens legen sich linienförmig gegen die unten liegende Seitenwand der beiden sich mit den offenen Seiten zugekehrten Führungsnuten. Das gesamte Körpergewicht wird dadurch beim linken oder rechten Auftreten des Fusses linienförmig vom Femurteil über den Gelenkzapfen auf den Tibiateil übertragen, so dass dadurch eine äusserst hohe Linienbelastung vom Gelenkzapfen auf die unten liegende Nutfläche mit erheblichem Verschleiss auftritt.

Die Aufgabe der Erfindung besteht darin, für die Herstellung der Verbindung zwischen Femur- und Tibiateil die Führungsnuten am hinteren, oben offenen Ende mit einem möglichst grossen Einlauf für die Enden des in die Führungsnuten durch Einhaken einzuführenden Gelenkzapfens zu versehen und in der Strecklage des Gelenkes bzw. des Unterschenkels zum Oberschenkel des Patienten die durch das Gewicht des Patienten entstehenden Kräfte gleichmässig vom Femur- auf den Tibiateil zu übertragen.

Diese Aufgabe wird bei der eingangs erwähnten Kniegelenk-Endoprothese dadurch gelöst, dass der Verlauf der unten liegenden Seitenflächen der beiden beliebig weiten Führungsnuten in Richtung ihrer geschlossenen Enden mit einem Flächenteil ansteigt und an diesen Enden in Zylinderflächen übergeht, deren Durchmesser gleich dem des Gelenkzapfens ist und gegen die die Enden des Gelenkzapfens in der Strecklage des Unterschenkels zum Oberschenkel etwa mit der halben Umfangsfläche anliegen.

Es kann damit die Weite der Führungsnuten und insbesondere deren Einlauf zur leichteren einhakenden Bewegung des Gelenkzapfens mit erleichterter Verbindung des Femur- und Tibiateiles beliebig gross gewählt werden, und trotzdem werden die Enden des Gelenkzapfens in der Strecklage in eine Flächenlage mit den Enden der Führungsnuten geführt, so dass damit die zu übertragenden Kräfte sehr günstig flächenförmig verteilt werden, womit ein Abrieb und ein Verschleiss auf ein Mindestmass herabgesetzt werden kann.

Die Erfindung wird nachstehend anhand der Zeichnung erläutert, in der ein Ausführungsbeispiel dargestellt ist.

Es zeigen:

Fig. 1 eine Aufsicht auf den Tibiateil einer Kniegelenk-Endoprothese,

Fig. 2 einen Schnitt nach Linie II-II der Fig. 1,

Fig. 3 eine Rückansicht einer Kniegelenk-Endoprothese mit in Eingriff befindlichem Femur- und Tibiateil.

Die Kniegelenk-Endoprothese besteht aus den mit einem Stiel 1 im Schienbein zu verankernden Tibiagleitflächen 2 aus Kunststoff beidseitig eines Mittelsteges 3 des Tibiateiles, in welchem ein Gelenkzapfen 4, z.B. entsprechend der Ausführung nach DE-A-2 549 819, gelagert ist. Die Enden des Gelenkzapfens 4, die nur Führungsaufgaben haben, greifen in mit ihren offenen Seiten einander zugekehrten Führungsnuten 5 der beiden Kufen 6 eines im Femurknochen mit einem Stiel 7 direkt oder indirekt zu verankernden Femurteiles. Die Führungsnuten 5 sind nach hinten und oben offen, wobei die Weite der Öffnungen 8 wesentlich grösser gewählt ist als der Durchmesser des Gelenkzapfens 4. Dadurch wird die Herstellung der Verbindung von Tibia- und Femurteil durch Einhaken der Enden des Zapfens 4 in die Führungsnuten 8, 5 wesentlich erleichtert.

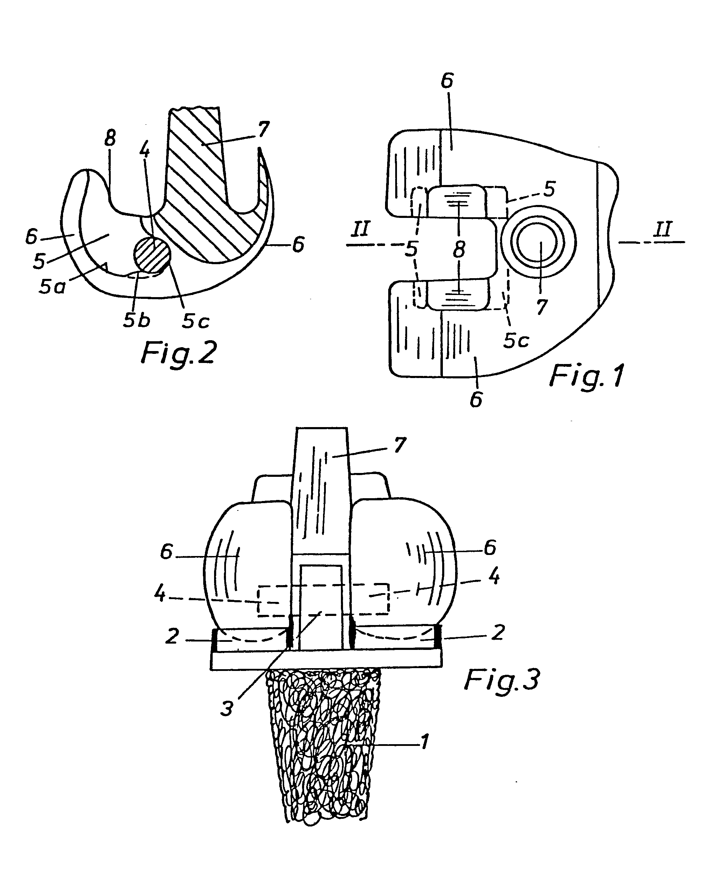

Weiter können die Führungsnuten 5 auch eine grosse Weite besitzen, wie in Fig. 2 gezeigt ist, so dass die Enden des Gelenkzapfens 4 eine grosse Bewegungsfreiheit besitzen, wie sie auch ein natürliches Kniegelenk aufweist. Die Wahl dieser grossen Weite ist zulässig, da der Gelenkzapfen 4 bei Gehbewegungen nur Führungsaufgaben zu erfüllen hat und nur in der Strecklage des Unterschenkels zum Oberschenkel die Kräfte durch das Gewicht des Patienten aufnehmen muss.

Zu diesem Zweckt steigt die untere Seitenfläche 5a gegenüber dem bekannten, gestrichelt angedeuteten Verlauf, mit dem Teil 5b vom tiefen Punkt wieder nach oben an und geht schliesslich am Ende der Führungsnuten 5 in Zylinderflächen 5c über, deren Durchmesser gleich dem des Gelenkzapfens 4 ist, der dadurch in der Strecklage flächenförmig etwa mit dem halben Umfang an die Zylinderflächen 5c zur Anlage kommt.

## Patentanspruch

Kniegelenk-Endoprothese, deren im Schienbein durch einen Stiel (1) verankerbaren Tibiateil Gleitflächen (2) beidseitig eines von vorn nach hinten verlaufenden Mittelsteges (3) besitzt, in welchem ein Gelenkzapfen (4) für den Femurteil gegenüber der Stielachse exzentrisch nach hinten im Mittelsteg versetzt gelagert ist und mit seinen Enden in mit den offenen Seiten einander zugekehrten Führungsnuten (5) der Kufen (6) des Femurteiles mit Spiel eingreift, welche Nuten (6) zur Einführung der beiden Enden des Gelenkzapfens (4) hinten nach oben offen enden, dadurch gekennzeichnet, dass der Verlauf der unten liegenden Seitenflächen (5a) der beiden beliebig weiten Führungsnuten (5) in Richtung ihrer geschlossenen Enden mit einem Flächenteil (5b) ansteigt und an diesen Enden in Zylinderflächen (5c) übergeht, deren Durchmesser gleich dem des Gelenkzapfens (4) ist und gegen die die Enden des Gelenkzapfens in der Strecklage des Unterschenkels zum Oberschenkel etwa mit der halben Umfangsfläche anliegen.

## Claim

Knee joint endoprosthesis of which the tibia portion, anchorable in the shin bone by means of a shank, has sliding surfaces (2) on each side of a central web (3) extending from front to back, in which a joint pin (4) for the femur portion is installed eccentrically offset rearwards in the central web with respect to the axis of the shank, and engages with its ends with play in guiding grooves (5) of the rockers (6) of the femur portion, whose open sides face towards each other, which grooves (6) are open ended towards the top at the back for insertion of the two ends of the joint pin (4), characterised in that the run of the underlaying lateral surfaces (5a) of the two guiding grooves (5) which are of any desired width rises with an areal portion (5b) in the direction towards their closed ends, and merges at these extremities into cylindrical surfaces (5c) the diameter of which is equal to that of the pivot pin (4) and against which the ends of the pivot pin bear with about half the peripheral surface in the extended position of the lower leg with respect to the thigh.

**Revendication**

Endoprothèse de l'articulation du genou, dont l'élément de tibia, qui peut être ancré au moyen d'une broche (1) dans le tibia, possède des surfaces de glissement (2) des deux côtés d'une branche médiane s'étendant d'avant en arrière et dans laquelle un pivot (4) pour l'élément de fémur est monté en étant excentré en arrière de la branche médiane par rapport à l'axe de la broche et s'engage, par ses extrémités, avec un certain jeu, dans des rainures de guidage (57, dont les faces ouvertes sont tournées l'une vers l'autre, des patins (6) de l'élément de fémur, lesquelles rainures (5) se terminent en étant ouvertes vers l'arrière et vers le bas pour l'introduction des deux extrémités du pivot (4), caractérisé par le fait que les surfaces latérales (5a), qui sont tournées vers le bas, des deux rainures de guidage (5) qui possèdent une largeur quelconque, remontent en direction de leurs extrémités fermées, sour la forme d'un élément de surface (5b) et se prolongent, au niveau de ces extrémités, par des surfaces cylindriques (5c), dont le diamètre est égal à celui du pivot (4) et contre lesquelles les extrémités du pivot s'appliquent approximativement sur la moitié de leur surface périphérique, lorsque le bas de la jambe est dans une position tendue par rapport à la cuisse.

0 178 445

Fig.2

8 4 7
6
5
5a
5b 5c

Fig.1

6
5
II II
5
8
7
5c
6

Fig.3

7
6 6
4 4
2 2
3
1

5